# EUROPEAN PATENT SPECIFICATION

(11) **EP 3 998 998 B1**
(45) Date of publication and mention of the grant of the patent: **07.06.2023**
(21) Application number: 20756961.7
(22) Date of filing: 17.07.2020
(51) Int. Cl.: A61F 2/38

(54) **CASE-LIKE DEVICE AND PROSTHETIC COMPONENT EQUIPPED WITH THIS DEVICE**
GERÄT MIT GEHÄUSE UND PROTHETISCHE KOMPONENTE, DIE MIT DIESEM GERÄT AUSGESTATTET IST
APPAREIL AVEC BOÎTIER ET COMPOSANT PROTHÉTIQUE ÉQUIPÉ DE CET APPAREIL

(30) Priority: 17.07.2019 IT 201900012201
(43) Date of publication of application: 25.05.2022
(73) Proprietor: Tecres S.P.A., 37066 Sommacampagna (VR) (IT)
(72) Inventor: SOFFIATTI, Renzo, 37066 Sommacampagna (Verona) (IT); FACCIOLI, Giovanni, 37066 Sommacampagna (Verona) (IT)
(74) Representative: Feltrinelli, Secondo Andrea
(86) International application number: PCT/IB2020/056743
(87) International publication number: WO 2021/009721

(56) References cited:
- EP-A1- 1 872 747
- EP-A1- 2 042 132
- US-A1- 2011 313 534
- US-B1- 7 070 622
- US-B2- 9 724 202

## Description

### TECHNICAL FIELD OF THE INVENTION

The present invention refers to a case-like device to be inserted under and/or around a prosthetic component, such as a tibial or femoral or humeral or elbow component of a permanent prosthesis, and in particular under the stem of the latter, in such a way that such case-like device, once assembled with the prosthetic component and/or the stem thereof, is suitable to be implanted at a bone of the patient such as the tibial or femoral bone of a knee joint, the femoral bone of a hip joint or the humeral bone of a shoulder joint, the elbow bone, etc..

Furthermore, the present invention refers also to a system or kit comprising a prosthetic component, such as for example a tibial and/or femoral component of a knee prosthesis, or a femoral component of a hip prosthesis or the humeral component of a shoulder prosthesis, or a component of an elbow prosthesis, equipped with a such case-like device.

### STATE OF THE PRIOR ART

As it is known, articular prostheses, after being implanted in the human body, despite being so-called "permanent" devices, may require their removal, in case, for example, of an infection arisen in the implantation site.

Another cause that may determine the removal of a prosthesis or of one of its components is that the fixed constraint of the connection between the same and the bones of the human body at which it has been implanted is lost.

Such an eventuality occurs, for example, with reference to the cemented knee prostheses, because the tibial component and/or the femoral component can "unstick" from the bone cement that is used for its connection to tibial bone.

The same applies to the femoral component of a hip prosthesis or for the humeral component of a shoulder prosthesis.

Furthermore, the direct contact between the metal with which the prosthesis is made and the patient's bone (for example in areas where the bone cement used to cement the prosthesis at the implant site has not arrived) can cause a phenomenon known as bone reabsorption (Stress Shielding). This phenomenon causes a real dissolution of the bone tissue with reduction of its cortical and trabecular mass, and the consequence is that the bone reduces its strength and the ability to withstand the loads transmitted thereto while the implanted prosthesis loses stability, detaches from the implant site and must be removed.

It is known indeed that a lasting result of a cemented implant depends on several factors, among which of main importance is the application way of the cement. The optimal cementation of a stem of a prosthesis foreseen to coat the same uniformly with a regular layer of approximately 2 mm of bone cement, so as to evenly distribute - by the layer of cement - the forces coming from the metal stem of the prosthesis.

If, however, the coating of cement has some gaps or windows, the metal stem can touch the bone with consequent - in few months - resorption of the bone portion in contact with the metal of the stem (Stress Shielding) and mobilization - and therefore failure - of the implant.

In conclusion, therefore, in order that a cemented prosthetic stem is perfectly affixed, it is necessary it possesses a regular, uniform, continuous cement mantle and having the correct thickness. However, these conditions are not easily obtainable by current cementation techniques. The positioning of the stem inside a diaphysis filled with cement takes place in fact "blindly" and the tip of the stem very often goes in touch with the bone, determining the serious consequences described above.

In the past, some attempts have been developed to equip the prosthetic stems with centering rings placed near the tip, but it is anyway a solution which is not entirely able to overcome the above-mentioned drawbacks.

All these disadvantages determine that the contact surfaces between prosthesis and bone cement, or as well as between bone and bone cement, are the weakest points of the implant and which more often cause the need to replace the permanent prosthesis.

The patent application EP1872747 discloses a hinged knee prosthesis.

The patent US7070622 discloses an articular prosthesis for knee, humerus or hip.

The patent application EP2042132 discloses a tibial prosthesis.

There is therefore the need for a solution that completely avoids any possible contact between the metal stem and the bone of the patient.

### OBJECTS OF THE INVENTION

A purpose of the present invention is to improve the state of the art.

Another purpose of the present invention is to provide a case-like device suitable to be positioned externally to a prosthetic component, and/or to the stem of a prosthetic component such as the tibial or femoral or humeral component of a prosthesis, and to ensure a layer of a biocompatible material (constituted by the device itself) to which the bone cement correctly adheres in a stable and long-lasting way.

A further purpose of the present invention is to provide a case-like device which ensures a cover layer of the metal stem of a prosthesis (different from the metal material with which the prosthetic stem is made) having an optimal minimum thickness on the whole surface of the stem itself.

A still further purpose of the present invention is to provide a kit composed of a prosthetic component such as a tibial and/or femoral component of a knee prosthesis, or a femoral component of a hip prosthesis o even the humeral component of a shoulder prosthesis, and a case-like device suitable to ensure a layer of a biocompatible material (different from the metal material with which the prosthesis is made) to which the bone cement correctly adheres in a stable way, with consequent stable and long-lasting constraint to the bone of the patient.

A further purpose of the present invention is to provide a kit wherein the prosthetic component equipped with a case-like device according to the present invention is suitable to completely avoid the risk of accidental contact between the prosthetic stem and the patient's bone.

According to one aspect of the present invention, a kit is provided equipped with a prosthetic component, possibly a tibial and/or femoral component of a knee prosthesis, or a femoral component of a hip prosthesis hi or a humeral component of a shoulder prosthesis, and with a case-like device according to the enclosed claim 1 .

According to another aspect of the present invention, a case-like device is provided according to the enclosed claim 17.

The dependent claims refer to preferred and advantageous embodiments of the invention.

### BRIEF DESCRIPTION OF THE DRAWINGS

Further characteristics and advantages of the present invention will be more evident from the detailed description of a preferred but not exclusive embodiment of a device for a prosthetic component such as a tibial component of a knee prosthesis, illustrated as a non-limiting example in the enclosed drawing tables in which:
figure 1 is a perspective view of a tibial component of a traditional type knee prosthesis;
figure 2 is a perspective view of a case-like device according to a version of the present invention;
figure 3 is perspective view slightly from below of a prosthetic component of a knee prosthesis assembled with the case-line device of figure 2;
figure 4 is a lateral view of the case-like device of figure 2;
figure 5 is a view from below of the case-like device of figure 2;
figure 6 is a lateral view of the case-like device of figure 2;
figure 7 is a sectional front view of the case-like device of figure 2, taken along the section plane A-A- of figure 6;
figure 8 is a perspective view of a femoral component of a hip prosthesis and of a case-like device according to a version of the present invention;
figure 9 is a perspective view slightly from above of a case-like device according of figure 8;
figure 10 is a rear view of a femoral component of a hip prosthesis and of the case-like device of figure 8, in an assembled version;
figure 11 is a lateral view of the prosthetic component of figure 8;
figure 12 is a lateral view of the assembled version of the femoral component of a hip prosthesis and of a case-like device of figure 10.

### EMBODIMENTS OF THE INVENTION

With reference to the enclosed figures, with 1 a component of a prosthesis is overall indicated.

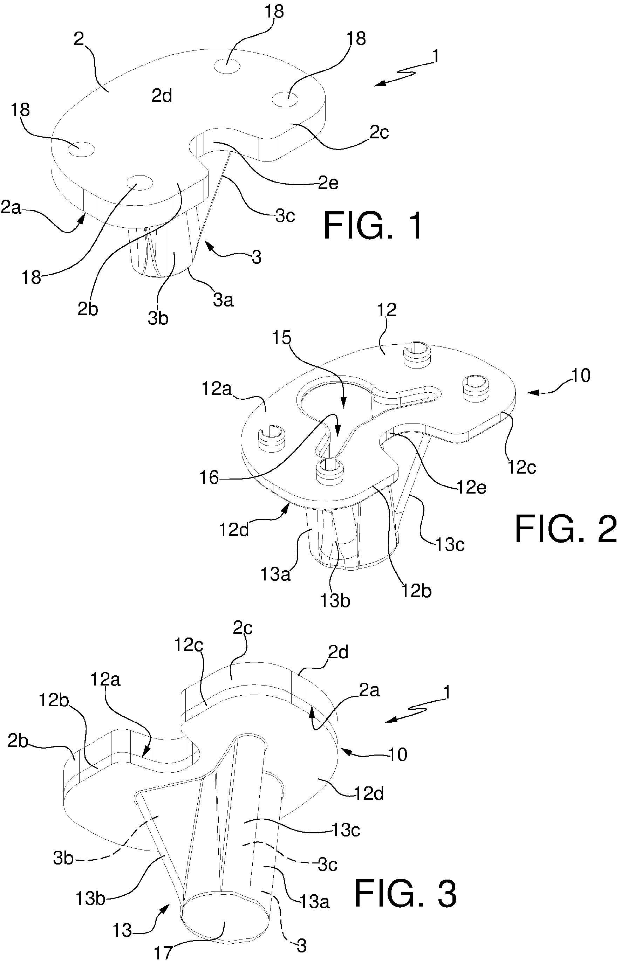

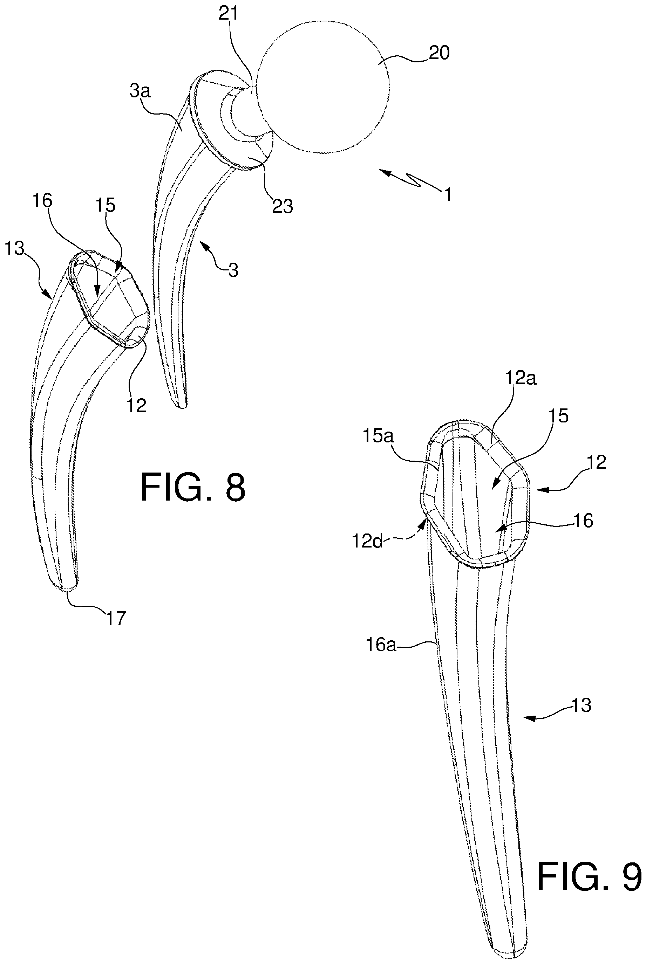

For example, the component 1 can be a tibial component of a prosthesis (for example total) of the knee, as shown in figures 1 to 7, and/or a femoral component of a knee prosthesis, and/or a femoral component of a hip prosthesis (as shown in figures 8 to 12) or yet a humeral component of a shoulder prosthesis (version not shown) or a component of a elbow prosthesis.

The prosthetic component 1, therefore, is a component of a prosthesis of an anatomical district of the human body equipped with a stem which in use is cemented to the bone of the patient.

Therefore, although in the description which follows and in the enclosed drawings respectively a tibial component of a knee prosthesis and a femoral component of a hip prosthesis will be described and shown, the present invention is to be considered valid also for other prosthetic components, to be implanted also in other districts of the human body with respect to the indicated joints.

With "proximal" in the present description it is meant a component or element closer to an end of a bone of a patient or closer to another component or element while the term "distal" it is meant a more inner component in a bone of a patient and/or farther away from one end of a bone of a patient or farther away from another component or element. The prosthetic component 1 is made by a stem 3.

Considering the case in which the prosthetic component 1 is a tibial component of a knee joint, it also includes a tibial plate 2, in addition to the stem 3.

The tibial plate 2 extends in a pattern substantially parallel to a transverse plane of the body human passing at the proximal end or proximal epiphysis of the tibia.

The tibial plate has a substantially C-shaped conformation, in which the two free ends 2b, 2c of the C are placed at the back of the knee.

The stem 3, in this version of the invention, has a portion 3a (or first portion 3 a) substantially cylindrical which extends from a lower face 2a of the tibial plate 2 in a substantially central position. The stem 3 and/or its portion 3a extend at the medullary canal of the patient's tibia.

The stem 3, in further versions, can have a substantially conical or frustoconical or pyramid frustum shape.

From the portion 3a at least two lateral wings 3b, 3c depart, having a substantially triangular shape, for example with a right angle. In particular, these lateral wings 3b, 3c each has one long side that adheres and/or is constrained substantially to the vertical extension in use of the portion 3a, a short side that adheres and/or is constrained substantially to the lower face 2a of the tibial plate 2 and at the end the diagonal which extends outwardly with respect to the portion 3a of the stem 3.

In particular, the short sides of the lateral wings 3b, 3c protrude toward the free ends 2b, 2c of the tibial plate 2. The angle enclosed between the lateral wings 3b, 3c (considering that subtended at the back portion of the knee) is less than 180° or better between 45° and 135°. However, this angle may vary according to the patient's needs.

The lateral wings, therefore, have respectively three vertices. Two vertices (those placed at the ends of the long side of the triangle) are connected and/or constrained to the portion 3a, one of these two vertices (the most proximal) also corresponds to one of the ends of the short side of the triangle. Therefore, the two vertices of each lateral wing which are placed at the ends of the short side of the triangle are connected and/or constrained to the lower face 2a of the tibial plate 2.

Therefore, one of those vertices is connected and/or constrained both to the portion 3a and to the lower face 2a of the tibial plate 2, being placed at the contact point between such two elements.

The tibial plate 2 also comprises an upper face 2d which faces toward the femoral component (not shown) of the knee prosthesis.

The present invention includes a case-like or shell-like device 10. The device 10 is suitable to externally cover at least the stem 3 of the prosthesis component 1. In the version illustrated in the figures 1 to 7, the device 10 covers also face the lower face 2a of the tibial plate 2 of the prosthetic component 1, which in such case is the tibial component of a knee prosthesis.

For this reason, in this version, the device 10 may be defined under-tibial device.

The device 10 is therefore able to house at least the stem 3 of the prosthetic component 1 inside it.

The device 10 therefore, in use, is an intermediate device that is positioned between the prosthetic component 1 and the patient's bone so that the latter is not in direct contact with the prosthetic component 1 at any point. In this way, since the prosthetic component 1 and/or the stem 3 is made of a metallic material while the device 10 is made of a biocompatible material other than metal, the risk of bone resorption is avoided and, at the same time, the prosthetic component 1 and/or stem 3 is constrained to the bone at the implant site.

The device 10 of the present invention therefore acts as a casing or shell that externally covers at least part of the prosthetic component 1 and/or at least the stem 3 of the prosthetic component 1.

Furthermore, the device 10 ensures the presence of a constant thickness along the entire surface of at least the stem 3 of the prosthetic component 1, so as to ensure a constant distance between the stem 3 and the patient's bone.

The prosthetic component 1, in fact, is made of a metal material while the device 10 is made of a biocompatible material different from the metal material of the prosthetic component 1.

In fact, therefore, the device 10 in use will be in contact with the bone interface of the implantation site, completely preventing that the contact with the latter occurs by the prosthetic component 1.

As regards the prosthetic component 1, therefore, the bone interface consists exclusively of the device 10, since the latter is interposed between the prosthetic component and the patient's bone.

Naturally, there will be some adhesion between the prosthetic component 1 and the device 10, also implemented by the bone cement in the liquid or fluid state used for cementing the prosthetic component itself, in order to determine a certain stability to the implant itself and avoid any play between the device 10 and the prosthetic component 1 (or better the stem 3).

The device 10 is made of a biocompatible plastic material, such as bone cement and/or PMMA, i.e. a material (solid) similar to bone cement in the liquid and/or viscous state that is traditionally used to bind the prostheses to the patient's bone. This affinity therefore allows perfect adhesion between the material of which the device 10 is made and the liquid and/or viscous bone cement used for bonding the prosthesis to the bone. The device 10 is however made of a material other than metal, in order to overcome the drawbacks mentioned above.

Furthermore, thanks to the fact that the device 10 already constitutes a layer of biocompatible material of the type indicated above, it requires a smaller quantity of cement needed to cement the stem 3 (it is in fact already pre-coated by the minimum optimal layer determined from the case-like device 10). Furthermore, this smaller amount of cement for implant fixation causes less thermal damage to the patient's bone, which usually to a greater or lesser extent is affected by contact with the bone cement in the liquid and/or fluid state and/or by temperatures which arise from its polymerization and hardening reaction.

Therefore, the device 10 has a surface wall having a thickness of at least 2 mm or between 0.5 mm and 5 mm.

Considering the version illustrated in figures 1 to 7, the device 10 comprises - in a way substantially corresponding to the conformation of the prosthetic component 1, when he latter is the tibial component of a knee prosthesis - a tibial base 12 having a substantially C-shaped form and equipped with an upper surface 12a and a lower surface 12d, as well as with two free ends of the C, 12b and 12c, facing the rear of the knee.

The tibial base 12 is adapted in use to come into contact and/or to be constrained with the lower face 2a of the tibial plate 2.

Therefore, the conformation and dimensions of the tibial base 12 substantially correspond to those of the tibial plate 2 (as visible in the assembled version of fig. 3). The tibial base 12 has a recess 12e which determines its C-shaped conformation thereof and which separates its free ends 12b and 12c. This recess corresponds to a recess 2e of the tibial plate 2 which performs the same function.

The device 10 also comprises a shaft 13.

In the version relating to the tibial component of a knee prosthesis, the shaft 13 is provided with a part 13a (or first part 13a) having a substantially tubular conformation. In particular, the part 13a has a side wall having a substantially tubular conformation inside which a first space 16a, for example cylindrical, is enclosed. Part 13a has a section (taken along a plane parallel to the transverse plane of the human body) which is substantially circular or oval, or square with rounded edges, or polygonal with rounded edges, etc.

The shaft 13 departs from the lower surface 12d of the tibial base 12, in a direction substantially perpendicular to the same, in order to enter the medullary canal of the patient's tibial bone.

Part 13a also includes at least two lateral protrusions 13b, 13c, having - in one version of the invention - a substantially triangular shape, for example at right angle. The lateral protrusions 13b, 13c have a conformation and an arrangement substantially corresponding to that of the lateral wings 3b, 3c, but of slightly larger dimensions, so as to be able to house the wings 3b, 3c inside them (and/or inside a respective space 16b, 16c having a conformation corresponding to that of the wings 3b, 3c).

The same applies to the part 13a of the shaft 13, which has slightly larger dimensions than those of the portion 3a of the stem 3, in order to be able to house the latter inside it. For this purpose, the device 10 according to the present invention comprises an opening 15. The opening 15 is an access opening that allows the insertion of the stem 3 of the prosthetic component 1 in the shaft 13 of the case-like or shell-like device 10.

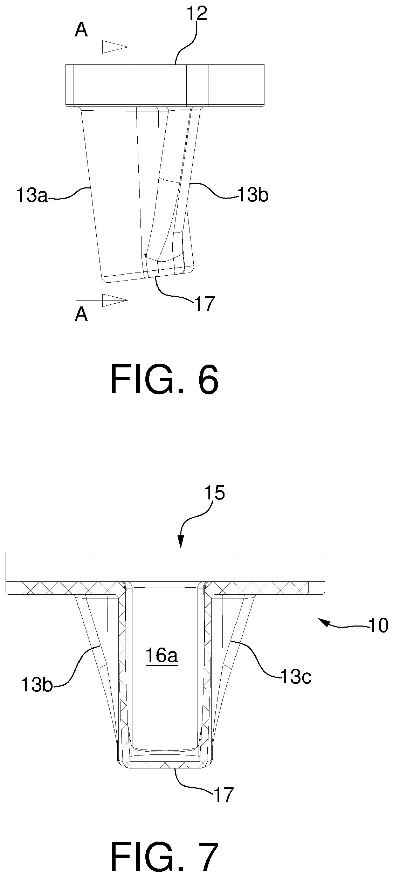

In particular, as visible in figures 7 and 9, the opening 15 subtends a cavity 16 present inside the device 10.

In the version in which the prosthetic component 1 is a tibial component of a knee prosthesis, the opening 15 affects at least the tibial base 12 and the cavity 16 affects at least the tibial base 12 and the part 13a of the shaft 13 of the device 10.

The opening 15, in general, comprises a zone 15a (or first zone 15a), having a substantially circular shape and dimensions slightly greater than those of the cross section of the first portion 3a of the stem 3, which in use is inserted inside the device 10 through the zone 15a.

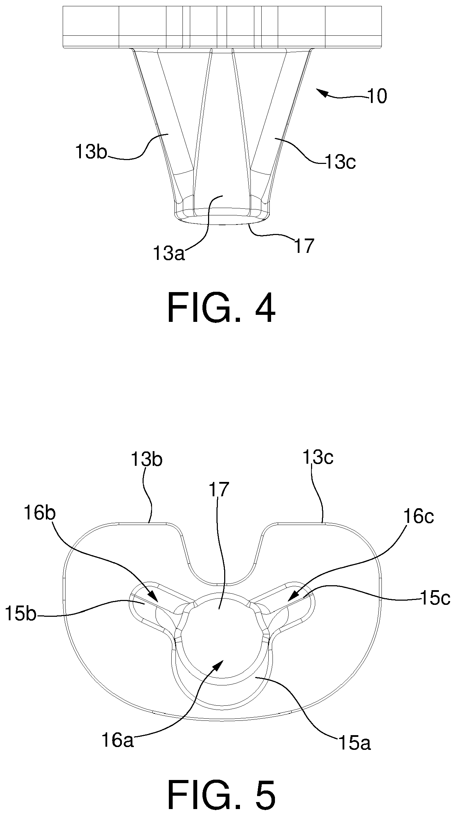

Considering the tibial version of the prosthetic component 1, the opening 15 also has at least two lateral openings 15b, 15c, visible for example in figure 5, which subtend respective hollow lateral portions suitable for inserting and housing in use the lateral wings 3b, 3b of the stem 3.

In this way, the structure is stiffened and is able to better withstand the loads to which it is subjected, and it is avoided that the prosthetic component 1 can rotate with respect to the device 10.

The lateral openings 15b, 15c have a lobed and/or elongated conformation, having a length equal to or slightly greater than the maximum transverse dimension of the lateral wings 13b, 13c and a width equal to or slightly greater than the thickness of the lateral wings 13b, 13c.

The cavity 16, therefore, at the zone 15a, has a space 16a, (or first space 16a) having a substantially cylindrical shape and slightly larger dimensions than those of the portion 3a which in use is housed inside the space 16a of the cavity 16.

In its inner part, the first space 16a has a substantially circular cross section, possibly corresponding to that of the zone 15a and/or of the portion 3a.

The cavity 16 also comprises, at the openings 15b, 15c, at least two lateral hollow portions 16b, 16c, which extend inside the lateral protrusions 13b, 13c of the shaft 13 of the device 10 according to the present invention, in the version in which the prosthetic component 1 is a tibial component of a knee prosthesis.

The hollow lateral portions 16b, 16c follow the course of the lateral protrusions 13b, 13c and of the lateral wings 3b, 3c and therefore become thinner, i.e. their width decreases, moving towards the distal end of the same.

Therefore, the lateral wings 3b, 3c, the lateral openings 15b, 15c, the at least two hollow lateral portions 16b, 16c (and possibly the lateral protrusions 13b, 13c) substantially have the same positions and configurations, but slightly different sizes, given that the lateral wings 3b, 3c in use are inserted inside the lateral openings 15b, 15c and are housed inside the at least two lateral hollow portions 16b, 16c, which in turn are positioned inside the lateral protrusions 13b, 13c.

As can be seen, for example, in figure 5, the opening 15 presents in plan view from above a substantially trilobed conformation, in which the lobes are formed by the first zone 15a and by the lateral openings 15b, 15c.

As can be seen in figures 5 and 6, the shaft 13 of the device 10 has a slightly inclined extension towards the rear of the knee. Furthermore, it can be seen the closing base 17 of the part 13a is rearwardly inclined upwards with respect to the tibial base 12 of the device itself.

The closing base 17 is placed in use in the innermost area of the medullary canal and constitutes the distal end of the shaft 13.

The closing base 17 can preferably be made of the same material that constitutes the device 10 and acts as a closure both for the part 13a of the shaft 13 and for the space 16a of the cavity 16.

This inclination naturally corresponds to that the stem 3 of the prosthetic component 1 may have, so as to allow its easy insertion inside the shaft 13 of the device 10.

The closing base 17, in at least one version of the present invention, is substantially perpendicular to the side wall of the part 13a of the shaft 13 while the side wall is inclined with respect to the tibial base 12 or better to its lower surface 12d.

In one version of the invention, the lateral protrusions 13b, 13c have a vertical extension (in use) slightly lower than that of the part 13a, and their terminal or distal zone, located near the base 17, is closed, not thus allowing the lateral hollow portions 16b, 16c to be opened at their distal end.

In one version of the invention, the lower surface 12d of the tibial base 12 can have roughness and/or undercuts and/or hollow cells, in order to better adhere and more effectively retain the bone cement which is used to constrain the whole to the patient's tibial bone.

Such roughness and/or undercuts and/or cells could also be present at the external wall of the shaft 13 of the device 10, with the same purposes.

In the version illustrated in figures 8 to 12, in which the prosthetic component 1 is a femoral component of a hip prosthesis, it is equipped with a stem 3 and a spherical or hemispherical or spherical cap head 20.

The stem 3 is designed to be inserted in use in the medullary canal of the bone, for example of the hip joint, while the head 20 is designed in use to articulate in the joint, for example in the acetabulum of the hip joint.

The stem 3 and the head 20 are connected by a neck structure 21, having for example a substantially cylindrical or truncated cone or pyramid shape.

The neck-like structure 21 has a first end 21a connected and/or constrained (in a fixed and/or adjustable way) to the head 20 and/or to a base present in the head 20.

The neck-like structure 21 also has a second end 21b, opposite to the first and connected and/or constrained, preferably in a fixed manner forming a single body, to the stem 3. The stem 3 has one end of connection 23, suitable in use to be connected and/or constrained to the neck 21, and a distal end 22, suitable in use to be inserted deep into the medullary canal of the bone.

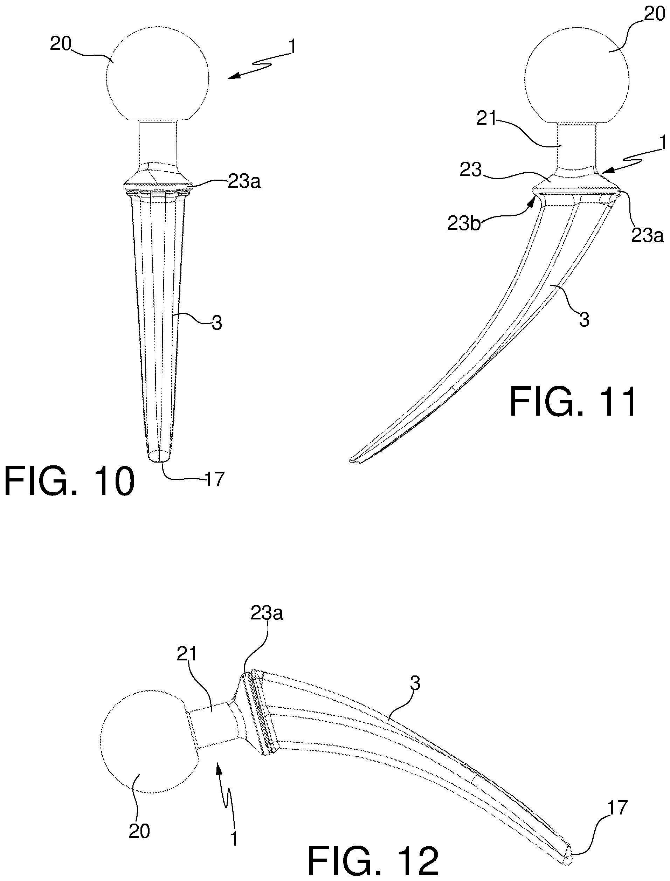

As visible for example from figure 11, the connection end 23 departs from the neck 21 outwards in all directions and therefore has a truncated cone or pyramid shape.

The connection end 23 then reaches with its maximum extension at the stem 3 itself, and/or at a portion 3a thereof, which is substantially tapered towards the distal end 22 and/or has a substantially conical or truncated cone or pyramid conformation.

At the junction between the connection end 23 and the stem 3 (and/or portion 3a), there is a perimetric flange 23a that extends outwards of the femoral component itself.

The perimeter flange 23a therefore has a greater lateral extension than that of the stem 3 and is therefore projecting externally with respect to the latter. There perimetric flange 23a therefore determines a lower face 23b illustrated for example in figure 11.

In this version of the invention, the device 10 has a shaft 13 equipped with a part 13a having a substantially tapered conformation towards the closing base 17 and/or having a substantially conical or truncated cone or pyramid conformation, i.e. substantially corresponding to the stem 3 but slightly larger in size, so as to be able to house the stem 3 itself and/or its portion 3a inside it.

At the opening 15 (and/or at the zone 15a of the opening 15), the device 10 has a flanged base 12 that extends outwards with respect to the opening 15.

This base 12 has an upper surface 12a and a lower surface 12d, opposite the upper surface 12a.

The base 12 has a lateral extension substantially corresponding to or slightly greater than the perimetric flange 23a of the stem 3.

The upper surface 12a is adapted in use to abut and/or to contact the perimetric flange 23a, and/or with its lower face 23b.

The lower surface 12d is suitable in use to abut and/or to come into contact and/or to be constrained to the patient's bone, thus completely avoiding contact between bone and material of the prosthetic component 1.

The conformation of the cavity 16 and/or of its space 16a corresponds to that of the shaft 13.

As can be seen from figures 11 and 12, the stem 3 of the prosthetic component 1 is laterally inclined and/or curved. Similarly, the device 10 has the same inclination and/or curvature.

The closing base 17 of the part 13a can therefore be inclined or not with respect to the wall of the part 13a of the shaft 13.

In one version of the invention, the device may have an internal metal core (not shown in the figures), which is completely immersed in the biocompatible material that constitutes the device 10 itself.

In an alternative version, the device 10 can be completely made of the materials indicated above, without providing an internal metal core.

Furthermore, the device 10 may or may not be impregnated with at least one active substance, such as an antibiotic or another medical agent, in order to treat an ongoing infection or other pathology in the surgical site.

According to a still further version, the device 10 can be porous and/or equipped with through holes, able to make the bone cement that is used to constrain the prosthetic component 1 to the patient's bone pass through them. In this case, the support function is not entrusted to the device 10, which therefore can have a high degree of holes and/or porosity. This function, in fact, is supported by the prosthetic component 1 itself.

In use, in fact, a very fluid bone cement can be used inside the device 10 and/or the cavity 16 (possibly inserted through the opening 15), which protrudes from the pores and/or through holes, constraining the device 10 to the patient's bone. In conjunction with this, the bone cement present inside the device 10 binds the latter to the prosthetic component 1.

Advantageously, the device 10 is preformed and/or formed of a rigid material, with predetermined sizes and thicknesses based on the type of prosthetic component 1 that must be housed.

In order to ensure an optimal connection of the device 10 to the prosthetic component 1, the latter may have housing seats 18 for corresponding connection means 19 which are present in the device 10, or vice versa.

In particular, as can be seen in figures 1 and 2, the housing seats 18 (four in the illustrated version, arranged at the outermost corners of the tibial plate 2 and/or two positioned at the front portion in use of the tibial plate 2 and other two substantially positioned at the free ends 2b, 2c, even if other positions or numbers can also be provided, especially for different conformations of the prosthetic component 1) are shaped as through openings arranged along an axis parallel to the longitudinal axis of the human body and/or in the vertical direction in use, in the thickness of the tibial plate 2. Alternatively, these housing seats 18 can be shaped as openings that are not through but open at the lower face 2a of the tibial plate 2.

The connection means 19, on the other hand, are housed inside the housing seats 18 (in this sense they correspond to and/or complement the latter) and can have a substantially pin, cylinder, semi-cylinder, clip, C, ring or tubular shape, etc. In particular, the connection means 19 are inserted inside the housing seats 18 and determine with the latter a constraint and/or a reference that allows adequate adhesion and/or constraint between the two involved components.

The cross section of the housing seats 18 is substantially corresponding and slightly larger than the cross section of the connection means 19, so that the latter can be housed (possibly in a removable way) inside the housing seats 18.

In the illustrated version, the housing seats 18 have a substantially circular cross section while the connection means 19 have a substantially C-shaped cross section.

In the illustrated version, the connection means 19 are positioned at the base 12 and/or at its upper surface 12a and/or protrude from the latter towards the prosthetic component.

As said, it would be possible to have the connection means 19 protruding from the lower face 2a of the tibial plate 2 and housing seats 18 whose opening is positioned at the upper surface 12a of the tibial base 12 and extending inside the latter.

In conclusion, the present invention refers, as indicated above, to a kit or system, or in any case to an assembly formed by prosthetic component 1 and case-like or shell-like device 10. In fact, in use, they are implanted together, in order to avoid - as mentioned - the contact of the prosthetic component material with the patient's bone, thanks to the interposition of the device 10.

The kit may also possibly include bone cement in a fluid or liquid state, which is used to cement the prosthetic component 1 to the device 10 and then to the patient's bone.

The invention thus conceived is susceptible of numerous modifications and variations, all falling within the scope of the inventive concept.

In addition, all details can be substituted by other technically equivalent elements. In practice, the materials used, as well as the contingent shapes and sizes, can be of any type in accordance with the requirements, without departing from the protective scope of the following claims

## Claims

1. Kit comprising a prosthetic component (1), provided with a stem (3), adapted to be implanted in use at a bone of a patient, such as for example a tibial and/or femoral component of a knee prosthesis adapted to be implanted at one end of the tibial bone and/or femoral bone of the patient at a knee joint, or a femoral component of a hip prosthesis, adapted to be implanted in use at one end of the femoral bone of the patient, or a humeral component of a shoulder prosthesis, or a component of an elbow prosthesis, and a case-like or shell-like device (10) provided with an opening (15) and with a cavity (16) and adapted to externally cover at least said stem (3) of said prosthetic component (1) and/or to house inside said cavity (16) at least said stem (3) of said prosthetic component (1), wherein said prosthetic component is made of a metal material, wherein said case-like or shell-like device (10) is in use placed between said prosthetic component (1) and the bone of the patient, in a manner such to prevent in use the direct contact between said prosthetic component (1) and the bone of the patient and to stably constrain said prosthetic component (1) to the bone of the patient, **characterized in that** said device (10) is made of a plastic material, such as bone cement and/or PMMA.

2. Kit according to claim 1, wherein said device (10) has a shape substantially corresponding to that of said prosthetic component (1) and/or of said stem (3), and/or wherein said prosthetic component (1) is a tibial component of a knee prosthesis and wherein said device (10) comprises a base (12) having substantially C-shaped form and provided with an upper surface (12a) and with a lower surface (12d), the latter being adapted to be constrained and/or to come into contact in use with the bone of the patient, as well as with two free ends (12b, 12c) of said substantially C-shaped form, directed in use towards the back of the knee or wherein said prosthetic component (1) is a femoral component of a hip prosthesis or a humeral component of a shoulder prosthesis or a component of an elbow prosthesis, and said device (10) comprises a flanged base (12) provided with an upper surface (12a) and with a lower surface (12d), the latter being adapted to be constrained and/or to come into contact in use with the bone of the patient.

3. Kit according to any one of the preceding claims, wherein said prosthetic component (1) comprises a tibial plate (2) and wherein said base (12) is adapted in use to come into contact and/or to be constrained with a lower face (2a) of said tibial plate (2) and/or has shape and size substantially corresponding to those of said tibial plate (2) and/or has a recess (12e) which determines said substantially C-shaped form and separates said free ends (12b, 12c).

4. Kit according to any one of the preceding claims, wherein said device (10) comprises a shaft (13) provided with a part (13a) having a substantially tubular or conical or frustoconical or pyramid frustum shape and a section taken along a plane parallel to the transverse plane of the human body that is substantially circular or oval, or square with smoothed edges, or polygonal with smoothed edges, etcetera, wherein said shaft (13) departs from a lower surface (12d) of said base (12), in a direction substantially perpendicular to said base (12) or tilted with respect to said base (12), so as to be inserted in the medullary canal of the bone of the patient and/or wherein said shaft (13) is in use adapted to house said stem (3) of said prosthetic component (1) and/or wherein said shaft (13) has dimensions slightly greater than those of said stem (3).

5. Kit according to any one of the preceding claims, wherein said prosthetic component has at least two lateral wings (3b, 3c) and wherein said part (13a) of said shaft (13) comprises at least two lateral protrusions (13b, 13c), possibly having a substantially triangular shape, for example right angle triangle, and/or a shape and a position substantially corresponding to that of said at least two lateral wings (3b, 3c) but with dimensions slightly greater than the size of said at least two lateral wings (3b, 3c), in a manner such that said at least two lateral protrusions (13b, 13c) can in use, at their interior, house said at least two lateral wings (3b, 3c).

6. Kit according to any one of the preceding claims, wherein said opening (15) is adapted to allow the insertion of said prosthetic component and/or of said stem (3) and/or of said at least two lateral wings (3b, 3c), wherein said opening (15) affects at least said base (12) and said part (13a) of said device (10).

7. Kit according to the preceding claim, wherein said opening (15) subtends said cavity (16) present in said device (10), wherein said opening (15) comprises a zone (15a), having substantially circular or polygonal shape and dimensions slightly greater than those of the transverse section of said portion (3a) of said stem (3) and/or at least two lateral openings (15b, 15c), adapted in use to insert said at least two lateral wings (3b, 3b) of said stem (3) and/or wherein said cavity (16) has, at said zone (15a), a space (16a), having substantially cylindrical or conical or frustoconical or pyramid frustum shape and dimensions slightly greater than those of said portion (3a) of said stem (3) which in use is housed in said space (16a) and/or, at said openings (15b, 15c), at least two lateral hollow portions (16b, 16c), which are extended within said lateral protrusions (13b, 13c) of said shaft (13) of said device (10).

8. Kit according to any one of the preceding claims, wherein said shaft (13) has in use a lateral progression tilted or curved or slightly tilted in use towards the back of the knee and/or comprises a closure base (17) of said part (13a) of said shaft (13), wherein said closure base (17) is tilted upward at the rear with respect to the base (12) of said device (10).

9. Kit according to any one of the preceding claims, wherein said device (10) is porous and/or provided with through holes, capable in use of moving bone cement from the interior of said device (10) to the exterior thereof and/or to the bone of the patient, such bone cement being possibly in liquid or fluid phase, suitable to anchor said prosthetic component (1) to the bone of the patient.

10. Kit according to any one of the preceding claims, wherein said prosthetic component (1) is said tibial component of a knee prosthesis and said tibial plate (2) is extended according to a progression substantially parallel to a transverse plane of the human body which passes at the proximal end or proximal epiphysis of the tibia and comprises said lower face (2a) and an upper face (2d), opposite said lower face (2a) and directed towards a femoral component of a knee prosthesis and/or wherein said tibial plate (2) has a substantially C-shaped form, with two free ends (2b, 2c) of said substantially C-shaped form in use placed at the back of the knee and a recess (2e) placed between said free ends (2b, 2c).

11. Kit according to any one of the preceding claims, wherein said stem (3) comprises a substantially cylindrical portion (3a) which is extended from said lower face (2a) of said tibial plate (2) and/or wherein said stem (3) and/or said portion (3a) are extended at the medullary canal of the tibia of the patient.

12. Kit according to any one of the preceding claims, wherein said stem (3) comprises at least two lateral wings (3b, 3c), wherein said at least two lateral wings (3b, 3c) depart from said portion (3a) and have a substantially triangular shape, for example with right angle and/or each have a long side that adheres and/or is substantially constrained to the vertical extension in use of said first portion (3a), a short side which adheres and/or is substantially constrained to said lower face (2a) of said tibial plate (2) and a diagonal which is extended outward with respect to said first portion (3a) of said stem (3).

13. Kit according to the preceding claim, wherein each short side of said at least two lateral wings (3b, 3c) is extended towards said free ends (2b, 2c) of said tibial plate (2) and/or wherein said at least two lateral wings (3b, 3c) determine an angle between them, in use at the rear part of the knee, less than 180° or comprised between 45° and 135°.

14. Kit according to any one of the preceding claims, wherein said prosthetic component (1) is a femoral component of a knee prosthesis or a humeral component of a shoulder prosthesis, or a component of an elbow prosthesis, wherein said prosthetic component comprises a spherical or semi-spherical or spherical cap-shaped head (20), and a neck-like structure (21) having a substantially cylindrical or frustoconical or pyramid frustum shape and adapted to connect said stem (3) and said head (20), wherein said stem (3) has a connection end (23), in use adapted to be connected and/or constrained to said neck-like structure (21), wherein said connection end (23) departs from said neck-like structure (21) towards the outside and has a frustoconical or pyramid frustum shape, up to said stem (3) where a perimeter flange (23a) is present which is extended towards the exterior of said prosthetic component (1) and provided with a lower face (23b).

15. Kit according to any one of the preceding claims, wherein said device (10) is preformed.

16. Kit according to any one of the preceding claims, wherein said prosthetic component (1) comprises housing seats (18) for corresponding connection means (19) present in said device (10), or vice versa, wherein said housing seats (18) are shaped as through openings or non-through openings which are extended along an axis parallel to the longitudinal axis of the human body and/or in use in a vertical direction in the thickness of said tibial plate (2) and provided with at least one opening at said lower face (2a) of said tibial plate (2) or of said perimeter flange (23a) while said connection means (19), in use adapted to be housed within said housing seats (18), have a substantially pin-like, cylinder-like, semi-cylinder, clip-like, C, ring or tubular shape, etcetera and are positioned at said base (12) and/or at said upper surface (12a) of said base (12).

17. Case-like or shell-like device (10) for a prosthetic component (1), such as for example a tibial and/or femoral component of a knee prosthesis adapted to be implanted at one end of the tibial bone and/or femoral bone of the patient at a knee joint, or a femoral component of a hip prosthesis, adapted to be implanted in use at one end of the femoral bone of the patient, or a humeral component of a shoulder prosthesis, or a component of an elbow prosthesis, wherein said prosthetic component is made of a metal material, wherein said prosthetic component according to one or more of claims 1 to 16, wherein said case-like or shell-like device (10) is suitable to be implanted in use at a bone of a patient, wherein said device (10) comprises an opening (15) and a cavity (16) and is in use adapted to externally cover and/or to house, at its interior, at least one stem (3) of said prosthetic component (1), wherein said device (10) is in use placed between said prosthetic component and the bone of the patient, in a manner so as to prevent in use the direct contact between the prosthetic component (1) and the bone of the patient and to stably constrain said prosthetic component to the bone of the patient, **characterized in that** said device (10) is made of a plastic material, such as bone cement and/or PMMA.

18. Device according to claim 17, wherein said device (10) has a shape substantially corresponding to that of said prosthetic component (1) and/or of said stem (3) of said prosthetic component (1), and/or wherein said device (10) comprises a base (12) having substantially C-shaped form and provided with an upper surface (12a) and with a lower surface (12d), as well as with two free ends (12b, 12c) of said substantially C-shaped form, in use directed towards the back of the knee, wherein said base (12) is in use adapted to come into contact and/or to be constrained with a lower face (2a) of said tibial plate (2) and/or has shape and size substantially corresponding to those of said tibial plate (2) and/or has a recess (12e) which determines said substantially C-shaped form and separates said free ends (12b, 12c) or wherein said device (10) comprises a flanged base (12) provided with an upper surface (12a) and with a lower surface (12d), the latter being adapted to be constrained to and/or to come into contact in use with the bone of the patient.

19. Device according to any one of claims 17 or 18, wherein said device (10) comprises a shaft (13) provided with a part (13a) having a substantially tubular or cylindrical or conical or frustoconical or pyramid frustum shape, and a section taken along a plane parallel to the transverse plane of the human body that is substantially circular or oval, or square with smoothed edges, or polygonal with smoothed edges, etcetera, wherein said shaft (13) departs from a lower surface (12d) of said base (12), in a direction substantially perpendicular or tilted with respect to said base (12), so as to be inserted in use in the medullary canal of the bone of the patient and/or wherein said shaft (13) is in use adapted to house said stem (3) of said prosthetic component (1) and/or wherein said shaft (13) has dimensions slightly greater than those of said stem (3).

20. Device according to any one of claims 17 to 19, wherein said part (13a) comprises at least two lateral protrusions (13b, 13c), possibly having a substantially triangular shape, for example right angled triangle, and/or a shape and a position that are substantially corresponding to that of at least two lateral wings (3b, 3c) of said prosthetic component (1) but with dimensions slightly greater than that of said at least two lateral wings (3b, 3c), in a manner such that said at least two lateral protrusions (13b, 13c) can in use house, at their interior, said at least two lateral wings (3b, 3c).

21. Device according to any one of claims 17 to 20, wherein said opening (15) is adapted to allow the insertion of said prosthetic component and/or of said stem (3) and/or of said at least two lateral wings (3b, 3c), wherein said opening (15) affects at least said base (12) and said part (13a) of said device (10), wherein said opening (15) subtends said cavity (16) present in said device (10), wherein said opening (15) comprises a zone (15a), having substantially circular shape and/or dimensions slightly greater than those of the transverse section of said portion (3a) and/or at least two lateral openings (15b, 15c), adapted in use to insert said at least two lateral wings (3b, 3b) of said stem (3) and/or wherein said cavity (16) has, at said zone (15a), a space (16a), having substantially cylindrical or conical or frustoconical or pyramid frustum shape and/or dimensions slightly greater than those of said portion (3a) which in use is housed in said space (16a) and/or, at said openings (15b, 15c), at least two lateral hollow portions (16b, 16c), which are extended within said lateral protrusions (13b, 13c) of said shaft (13) of said device (10).

22. Device according to any one of claims 17 to 21, wherein said shaft (13) has a slightly tilted lateral progression, possibly in use tilted towards the back of the knee, and comprises a closure base (17) of said part (13a), wherein said closure base (17) is tilted, on the rear part, upward with respect to the base (12) of said device (10) and/or wherein said device (10) is porous and/or provided with through holes, capable in use of moving bone cement from the interior of said device (10) to the exterior thereof and/or to the bone of the patient, such bone cement possibly in liquid or fluid phase, adapted to anchor said prosthetic component (1) to the bone of the patient.

## Patentansprüche

1. Bausatz, umfassend eine mit einem Stiel (3) versehene Prothesenkomponente (1), die dazu ausgelegt ist, bei Verwendung an einem Knochen eines Patienten implantiert zu werden, wie beispielsweise eine Tibia- und/oder Femurkomponente einer Knieprothese, die dazu ausgelegt ist, an einem Ende des Tibiaknochens und/oder Femurknochens des Patienten an einem Kniegelenk implantiert zu werden, oder eine Femurkomponente einer Hüftprothese, die dazu ausgelegt ist, bei Verwendung an einem Ende des Femurknochens des Patienten implantiert zu werden, oder eine Humeruskomponente einer Schulterprothese, oder eine Komponente einer Ellbogenprothese, und eine gehäuseartige oder schalenartige Vorrichtung (10), die mit einer Öffnung (15) und mit einer Kavität (16) versehen ist und dazu ausgelegt ist, mindestens den besagten Stiel (3) der besagten Prothesenkomponente (1) äußerlich abzudecken und/oder im Inneren der besagten Kavität (16) mindestens den besagten Stiel (3) der besagten Prothesenkomponente (1) aufzunehmen, worin die besagte Prothesenkomponente aus einem Metallmaterial hergestellt ist, worin die besagte gehäuseartige oder schalenartige Vorrichtung (10) bei Verwendung zwischen der besagten Prothesenkomponente (1) und dem Knochen des Patienten in einer solchen Weise angeordnet ist, um bei Verwendung den direkten Kontakt zwischen der besagten Prothesenkomponente (1) und dem Knochen des Patienten zu verhindern und die besagte Prothesenkomponente (1) stabil an den Knochen des Patienten zu binden, **dadurch gekennzeichnet, dass** die besagte Vorrichtung (10) aus einem Kunststoffmaterial, wie Knochenzement und/oder PMMA, hergestellt ist.

2. Bausatz nach Anspruch 1, worin die besagte Vorrichtung (10) eine Form aufweist, die im Wesentlichen derjenigen der besagten Prothesenkomponente (1) und/oder des besagten Stiels (3) entspricht, und/oder worin die besagte Prothesenkomponente (1) eine Tibiakomponente einer Knieprothese ist und worin die besagte Vorrichtung (10) eine Basis (12) umfasst, die eine im Wesentlichen C-förmige Form aufweist und mit einer oberen Oberfläche (12a) und mit einer unteren Oberfläche (12d) versehen ist, wobei letztere dazu ausgelegt ist, bei Verwendung an den Knochen des Patienten gebunden zu werden und/oder damit in Kontakt zu kommen, sowie mit zwei freien Enden (12b, 12c) der besagten im Wesentlichen C-förmigen Form, die bei Verwendung zur Rückseite des Knies hin gerichtet sind, oder worin die besagte Prothesenkomponente (1) eine Femurkomponente einer Hüftprothese oder eine Humeruskomponente einer Schulterprothese oder eine Komponente einer Ellbogenprothese ist, und die besagte Vorrichtung (10) eine geflanschte Basis (12) umfasst, die mit einer oberen Oberfläche (12a) und mit einer unteren Oberfläche (12d) versehen ist, wobei letztere dazu ausgelegt ist, bei Verwendung an den Knochen des Patienten gebunden zu werden und/oder damit in Kontakt zu kommen.

3. Bausatz nach irgendeinem der vorangegangenen Ansprüche, worin die besagte Prothesenkomponente (1) eine Tibiaplatte (2) umfasst und worin die besagte Basis (12) bei Verwendung dazu ausgelegt ist, mit einer Unterseite (2a) der besagten Tibiaplatte (2) in Kontakt zu kommen und/oder daran gebunden zu werden und/oder eine Form und Größe aufweist, die im Wesentlichen derjenigen der besagten Tibiaplatte (2) entspricht, und/oder eine Aussparung (12e) aufweist, die die besagte im Wesentlichen C-förmige Form bestimmt und die besagten freien Enden (12b, 12c) trennt.

4. Bausatz nach irgendeinem der vorangegangenen Ansprüche, worin die besagte Vorrichtung (10) einen Schaft (13) umfasst, der mit einem Teil (13a) versehen ist, der eine im Wesentlichen rohrförmige oder konische oder kegelstumpfförmige oder pyramidenstumpfförmine Form und einen Querschnitt entlang einer Ebene parallel zur Querebene des menschlichen Körpers aufweist, der im Wesentlichen kreisförmig oder oval oder quadratisch mit abgerundeten Kanten oder polygonal mit abgerundeten Kanten usw. ist, worin der besagte Schaft (13) von einer unteren Oberfläche (12d) der besagten Basis (12) in eine Richtung ausgeht, die im Wesentlichen senkrecht zur besagten Basis (12) oder geneigt in Bezug auf die besagte Basis (12) ist, sodass er in den Markkanal des Knochens des Patienten eingeführt werden kann und/oder worin der besagte Schaft (13) bei Verwendung dazu ausgelegt ist, den besagten Stiel (3) der besagten Prothesenkomponente (1) aufzunehmen und/oder worin der besagte Schaft (13) Abmessungen aufweist, die geringfügig größer sind als diejenigen des besagten Stiels (3) .

5. Bausatz nach irgendeinem der vorangegangenen Ansprüche, worin die besagte Prothesenkomponente mindestens zwei seitliche Flügel (3b, 3c) aufweist und worin der besagte Teil (13a) des besagten Schafts (13) mindestens zwei seitliche Vorsprünge (13b, 13c) umfasst, die möglichst eine im Wesentlichen dreieckige Form, beispielsweise rechtwinkliges Dreieck, aufweisen und/oder eine Form und eine Position, die im Wesentlichen derjenigen der besagten mindestens zwei seitlichen Flügel (3b, 3c) entspricht, aber mit Abmessungen, die geringfügig größer sind als die Größe der besagten mindestens zwei seitlichen Flügel (3b, 3c), in einer solchen Weise, dass die besagten mindestens zwei seitlichen Vorsprünge (13b, 13c) bei Verwendung die besagten mindestens zwei seitlichen Flügel (3b, 3c) in ihrem Inneren aufnehmen können.

6. Bausatz nach irgendeinem der vorangegangenen Ansprüche, worin die besagte Öffnung (15) dazu ausgelegt ist, die Einführung der besagten Prothesenkomponente und/oder des besagten Stiels (3) und/oder der besagten mindestens zwei seitlichen Flügel (3b, 3c) zu ermöglichen, worin die besagte Öffnung (15) mindestens die besagte Basis (12) und den besagten Teil (13a) der besagten Vorrichtung (10) beeinflusst.

7. Bausatz nach dem vorangegangenen Anspruch, worin die besagte Öffnung (15) die besagte Kavität (16) einschließt, die in der besagten Vorrichtung (10) vorhanden ist, worin die besagte Öffnung (15) eine Zone (15a) umfasst, mit im Wesentlichen kreisförmiger oder polygonaler Form und Abmessungen, die geringfügig größer sind als diejenigen des Querschnitts des besagten Abschnitts (3a) des besagten Stiels (3) und/oder mindestens zwei seitliche Öffnungen (15b, 15c), die bei Verwendung zum Einführen der besagten mindestens zwei seitlichen Flügel (3b, 3b) des besagten Schafts (3) ausgelegt sind, und/oder worin die besagte Kavität (16) an der besagten Zone (15a) einen Raum (16a) aufweist, mit im Wesentlichen zylindrischer oder konischer oder kegelstumpfförmiger oder pyramidenstumpfförmiger Form und Abmessungen, die geringfügig größer sind als diejenigen des besagten Abschnitts (3a) des besagten Stiels (3), der bei Verwendung in dem besagten Raum (16a) untergebracht ist, und/oder, an den Öffnungen (15b, 15c), mindestens zwei seitliche hohle Abschnitte (16b, 16c), die sich innerhalb der besagten seitlichen Vorsprünge (13b, 13c) des besagten Schafts (13) der besagten Vorrichtung (10) erstrecken.

8. Bausatz nach irgendeinem der vorangegangenen Ansprüche, worin der besagte Schaft (13) bei Verwendung einen seitlichen Verlauf aufweist, der zur Rückseite des Knies hin geneigt oder gekrümmt oder leicht geneigt ist, und/oder eine Verschlussbasis (17) des besagten Teils (13a) des besagten Schafts (13) umfasst, worin die besagte Verschlussbasis (17) in Bezug auf die Basis (12) der besagten Vorrichtung (10) an der Rückseite nach oben geneigt ist.

9. Bausatz nach irgendeinem der vorangegangenen Ansprüche, worin die besagte Vorrichtung (10) porös und/oder mit Durchgangslöchern versehen ist, die bei Verwendung fähig sind, Knochenzement aus dem Inneren der besagten Vorrichtung (10) zu ihrer Außenseite und/oder zum Knochen des Patienten zu bewegen, wobei dieser Knochenzement möglichst in Flüssig- oder Fluidphase vorliegt, die dazu geeignet ist, die besagte Prothesenkomponente (1) am Knochen des Patienten zu verankern.

10. Bausatz nach irgendeinem der vorangegangenen Ansprüche, worin die besagte Prothesenkomponente (1) die besagte Tibiakomponente einer Knieprothese ist und die besagte Tibiaplatte (2) sich gemäß einem Verlauf im Wesentlichen parallel zu einer Querebene des menschlichen Körpers erstreckt, die am proximalen Ende oder der proximalen Epiphyse der Tibia verläuft und die besagte Unterseite (2a) und eine Oberseite (2d), die der besagten Unterseite (2a) gegenüberliegt und zu einer Femurkomponente einer Knieprothese hin gerichtet ist, umfasst und/oder worin die besagte Tibiaplatte (2) eine im Wesentlichen C-förmige Form aufweist, wobei zwei freie Enden (2b, 2c) der im Wesentlichen C-förmigen Form bei Verwendung an der Rückseite des Knies angeordnet sind und eine Aussparung (2e) zwischen den besagten freien Enden (2b, 2c) angeordnet ist.

11. Bausatz nach irgendeinem der vorangegangenen Ansprüche, worin der besagte Stiel (3) einen im Wesentlichen zylindrischen Abschnitt (3a) umfasst, der sich von der besagten Unterseite (2a) der besagten Tibiaplatte (2) aus erstreckt und/oder worin der besagte Stiel (3) und/oder der besagte Abschnitt (3a) sich am Markkanal der Tibia des Patienten erstreckt.

12. Bausatz nach irgendeinem der vorangegangenen Ansprüche, worin der besagte Stiel (3) mindestens zwei seitliche Flügel (3b, 3c) umfasst, worin die besagten mindestens zwei seitlichen Flügel (3b, 3c) von dem besagten Abschnitt (3a) ausgehen und eine im Wesentlichen dreieckige Form, beispielsweise mit rechtem Winkel, aufweisen und/oder jeweils eine lange Seite, die an der vertikalen Erstreckung bei Verwendung des besagten ersten Abschnitts (3a) anhaftet und/oder im Wesentlichen daran gebunden ist, eine kurze Seite, die an der besagten Unterseite (2a) der besagten Tibiaplatte (2) anhaftet und/oder im Wesentlichen daran gebunden ist, und eine Diagonale, die sich in Bezug auf den besagten ersten Abschnitt (3a) des besagten Stiels (3) nach außen erstreckt, aufweisen.

13. Bausatz nach dem vorangegangenen Anspruch, worin jede kurze Seite der besagten mindestens zwei seitlichen Flügel (3b, 3c) sich zu den besagten freien Enden (2b, 2c) der besagten Tibiaplatte (2) hin erstreckt und/oder worin die besagten mindestens zwei seitlichen Flügel (3b, 3c) bei Verwendung am hinteren Teil des Knies einen Winkel zwischen ihnen bestimmen, der kleiner als 180° ist oder zwischen 45° und 135° liegt.

14. Bausatz nach irgendeinem der vorangegangenen Ansprüche, worin die besagte Prothesenkomponente (1) eine Femurkomponente einer Knieprothese oder eine Humeruskomponente einer Schulterprothese oder eine Komponente einer Ellbogenprothese ist, worin die besagte Prothesenkomponente einen kugelförmigen oder halbkugelförmigen oder kugelförmigen kappenförmigen Kopf (20) und eine halsartige Struktur (21) mit einer im Wesentlichen zylindrischen oder kegelstumpfförmigen oder pyramidenstumpfförmigen Form umfasst und dazu ausgelegt ist, den besagten Stiel (3) und den besagten Kopf (20) zu verbinden, worin der besagte Stiel (3) ein Verbindungsende (23) aufweist, das bei Verwendung dazu ausgelegt ist, mit der besagten halsartigen Struktur (21) verbunden und/oder daran gebunden zu werden, worin das besagte Verbindungsende (23) von der besagten halsartigen Struktur (21) nach außen hin ausgeht und eine kegelstumpfförmige oder pyramidenstumpfförmige Form aufweist, bis hin zu dem besagten Stiel (3), an dem ein Umfangsflansch (23a) vorhanden ist, der sich zur Außenseite der besagten Prothesenkomponente (1) hin erstreckt und mit einer Unterseite (23b) versehen ist.

15. Bausatz nach irgendeinem der vorangegangenen Ansprüche, worin die besagte Vorrichtung (10) vorgeformt wird.

16. Bausatz nach irgendeinem der vorangegangenen Ansprüche, worin die besagte Prothesenkomponente (1) Gehäusesitze (18) für entsprechende in der Vorrichtung (10) vorhandene Verbindungsmittel (19) umfasst, oder umgekehrt, worin die besagten Gehäusesitze (18) als Durchgangsöffnungen oder Nicht-Durchgangsöffnungen geformt sind, die sich entlang einer Achse parallel zur Längsachse des menschlichen Körpers und/oder bei Verwendung in einer vertikalen Richtung in der Dicke der besagten Tibiaplatte (2) erstrecken und mit mindestens einer Öffnung an der besagten Unterseite (2a) der besagten Tibiaplatte (2) oder des besagten Umfangsflansches (23a) versehen sind, während die besagten Verbindungsmittel (19), die bei Verwendung dazu ausgelegt sind, in den besagten Gehäusesitzen (18) untergebracht zu werden, eine im Wesentlichen stiftartige, zylinderartige, halbzylindrische, klammerartige, C-, Ring- oder rohrförmige Form usw. aufweisen und an der besagten Basis (12) und/oder an der besagten oberen Oberfläche (12a) der besagten Basis (12) angeordnet sind.

17. Gehäuseartige oder schalenartige Vorrichtung (10) für eine Prothesenkomponente (1), wie beispielsweise eine Tibia- und/oder Femurkomponente einer Knieprothese, die dazu ausgelegt ist, an einem Ende des Tibiaknochens und/oder Femurknochens des Patienten an einem Kniegelenk implantiert zu werden, oder eine Femurkomponente einer Hüftprothese, die dazu ausgelegt ist, bei Verwendung an einem Ende des Femurknochens des Patienten implantiert zu werden, oder eine Humeruskomponente einer Schulterprothese, oder eine Komponente einer Ellbogenprothese, worin die besagte Prothesenkomponente aus einem Metallmaterial hergestellt ist, worin die besagte Prothesenkomponente einem oder mehreren der Ansprüche 1 bis 16 entspricht, worin die besagte gehäuseartige oder schalenartige Vorrichtung (10) geeignet ist, bei Verwendung an einem Knochen eines Patienten implantiert zu werden, worin die besagte Vorrichtung (10) eine Öffnung (15) und eine Kavität (16) umfasst und bei Verwendung dazu ausgelegt ist, mindestens einen Stiel (3) der besagten Prothesenkomponente (1) äußerlich abzudecken und/oder in ihrem Inneren aufzunehmen, worin die besagte Vorrichtung (10) bei Verwendung zwischen der besagten Prothesenkomponente und dem Knochen des Patienten in einer solchen Weise angeordnet ist, um bei Verwendung den direkten Kontakt zwischen der besagten Prothesenkomponente (1) und dem Knochen des Patienten zu verhindern und die besagte Prothesenkomponente (1) stabil an den Knochen des Patienten zu binden, **dadurch gekennzeichnet, dass** die besagte Vorrichtung (10) aus einem Kunststoffmaterial, wie Knochenzement und/oder PMMA, hergestellt ist.

18. Vorrichtung nach Anspruch 17, worin die besagte Vorrichtung (10) eine Form aufweist, die im Wesentlichen derjenigen der besagten Prothesenkomponente (1) und/oder des besagten Stiels (3) der besagten Prothesenkomponente (1) entspricht, und/oder worin die besagte Vorrichtung (10) eine Basis (12) umfasst, die eine im Wesentlichen C-förmige Form aufweist und mit einer oberen Oberfläche (12a) und mit einer unteren Oberfläche (12d) sowie mit zwei freien Enden (12b, 12c) der besagten im Wesentlichen C-förmigen Form versehen ist, die bei Verwendung zur Rückseite des Knies hin gerichtet sind, worin die besagte Basis (12) bei Verwendung dazu ausgelegt ist, mit einer Unterseite (2a) der besagten Tibiaplatte (2) in Kontakt zu kommen und/oder daran gebunden zu werden und/oder eine Form und Größe aufweist, die im Wesentlichen derjenigen der besagten Tibiaplatte (2) entspricht, und/oder eine Aussparung (12e) aufweist, die die besagte im Wesentlichen C-förmige Form bestimmt und die besagten freien Enden (12b, 12c) trennt oder worin die besagte Vorrichtung (10) eine geflanschte Basis (12) umfasst, die mit einer oberen Oberfläche (12a) und mit einer unteren Oberfläche (12d) versehen ist, wobei letztere dazu ausgelegt ist, bei Verwendung an den Knochen des Patienten gebunden zu werden und/oder damit in Kontakt zu kommen.

19. Vorrichtung nach irgendeinem der Ansprüche, 17 oder 18, worin die besagte Vorrichtung (10) einen Schaft (13) umfasst, der mit einem Teil (13a) versehen ist, der eine im Wesentlichen rohrförmige oder zylindrische oder konische oder kegelstumpfförmige oder pyramidenstumpfförmine Form und einen Querschnitt entlang einer Ebene parallel zur Querebene des menschlichen Körpers aufweist, der im Wesentlichen kreisförmig oder oval oder quadratisch mit abgerundeten Kanten oder polygonal mit abgerundeten Kanten usw. ist, worin der besagte Schaft (13) von einer unteren Oberfläche (12d) der besagten Basis (12) in eine Richtung ausgeht, die im Wesentlichen senkrecht oder geneigt in Bezug auf die besagte Basis (12) ist, sodass er bei Verwendung in den Markkanal des Knochens des Patienten eingeführt werden kann und/oder worin der besagte Schaft (13) bei Verwendung dazu ausgelegt ist, den besagten Stiel (3) der besagten Prothesenkomponente (1) aufzunehmen und/oder worin der besagte Schaft (13) Abmessungen aufweist, die geringfügig größer sind als diejenigen des besagten Stiels (3).

20. Vorrichtung nach irgendeinem der Ansprüche 17 bis 19, worin der besagte Teil (13a) mindestens zwei seitliche Vorsprünge (13b, 13c) umfasst, die möglichst eine im Wesentlichen dreieckige Form, beispielsweise rechtwinkliges Dreieck, aufweisen und/oder eine Form und eine Position, die im Wesentlichen derjenigen der mindestens zwei seitlichen Flügel (3b, 3c) der besagten Prothesenkomponente (1) entspricht, aber mit Abmessungen, die geringfügig größer als die der besagten mindestens zwei seitlichen Flügel (3b, 3c) sind, in einer solchen Weise, dass die besagten mindestens zwei seitlichen Vorsprünge (13b, 13c) bei Verwendung die besagten mindestens zwei seitlichen Flügel (3b, 3c) in ihrem Inneren aufnehmen können.

21. Vorrichtung nach irgendeinem der Ansprüche 17 bis 20, worin die besagte Öffnung (15) dazu ausgelegt ist, die Einführung der besagten Prothesenkomponente und/oder des besagten Stiels (3) und/oder der besagten mindestens zwei seitlichen Flügel (3b, 3c) zu ermöglichen, worin die besagte Öffnung (15) mindestens die besagte Basis (12) und den besagten Teil (13a) der besagten Vorrichtung (10) beeinflusst, worin die besagte Öffnung (15) die besagte Kavität (16) einschließt, die in der besagten Vorrichtung (10) vorhanden ist, worin die besagte Öffnung (15) eine Zone (15a) umfasst, mit im Wesentlichen kreisförmiger Form und/oder Abmessungen, die geringfügig größer sind als diejenigen des Querschnitts des besagten Abschnitts (3a) und/oder mindestens zwei seitliche Öffnungen (15b, 15c), die bei Verwendung zum Einführen der besagten mindestens zwei seitlichen Flügel (3b, 3b) des besagten Stiels (3) ausgelegt sind, und/oder worin die besagte Kavität (16) an der besagten Zone (15a) einen Raum (16a) aufweist, mit im Wesentlichen zylindrischer oder konischer oder kegelstumpfförmiger oder pyramidenstumpfförmiger Form und/oder Abmessungen, die geringfügig größer sind als diejenigen des besagten Abschnitts (3a), der bei Verwendung in dem besagten Raum (16a) untergebracht ist, und/oder, an den besagten Öffnungen (15b, 15c), mindestens zwei seitliche hohle Abschnitte (16b, 16c), die sich innerhalb der besagten seitlichen Vorsprünge (13b, 13c) des besagten Schafts (13) der besagten Vorrichtung (10) erstrecken.

22. Vorrichtung nach einem der Ansprüche 17 bis 21, **dadurch gekennzeichnet, dass** der Schaft (13) einen leicht geneigten seitlichen Verlauf aufweist, der bei Verwendung möglichst zur Rückseite des Knies hin geneigt ist, und eine Verschlussbasis (17) des besagten Teils (13a) umfasst, worin die besagte Verschlussbasis (17) am hinteren Teil in Bezug auf die Basis (12) der besagten Vorrichtung (10) nach oben geneigt ist und/oder worin die besagte Vorrichtung (10) porös und/oder mit Durchgangslöchern versehen ist, die bei Verwendung fähig sind, Knochenzement aus dem Inneren der besagten Vorrichtung (10) zu ihrer Außenseite und/oder zum Knochen des Patienten zu bewegen, wobei dieser Knochenzement möglichst in Flüssig- oder Fluidphase vorliegt, die dazu geeignet ist, die besagte Prothesenkomponente (1) am Knochen des Patienten zu verankern.

## Revendications

1. Kit comprenant un composant prosthétique (1), doté d'une tige (3), adapté pour être implanté pendant l'utilisation sur un os d'un patient, comme par exemple un composant tibial et/ou fémoral d'une prothèse de genou adaptée pour être implantée à une extrémité de l'os tibial et/ou de l'os fémoral du patient sur une articulation de genou, ou un composant fémoral d'une prothèse de hanche, adaptée pour être implantée pendant l'utilisation à une extrémité de l'os fémoral du patient, ou un composant huméral d'une prothèse d'épaule, ou un composant d'une prothèse de coude, et un dispositif en forme de boîtier ou de coque (10) doté d'une ouverture (15) et d'une cavité (16) et adapté pour recouvrir extérieurement au moins ladite tige (3) dudit composant prosthétique (1) et/ou pour contenir à l'intérieur de ladite cavité (16) au moins ladite tige (3) dudit composant prosthétique (1), dans lequel ledit composant prosthétique est constitué d'un matériau métallique, dans lequel ledit dispositif en forme de boîtier ou de coque (10) est placé pendant l'utilisation entre ledit composant prosthétique (1) et l'os du patient, de manière à empêcher pendant l'utilisation le contact direct entre ledit composant prosthétique (1) et l'os du patient et à maintenir de manière stable ledit composant prosthétique (1) sur l'os du patient, **caractérisé en ce que** ledit dispositif (10) est constitué d'une matière plastique, comme le ciment osseux et/ou PMMA.

2. Kit selon la revendication 1, dans lequel ledit dispositif (10) a une forme correspondant sensiblement à celle dudit composant prosthétique (1) et/ou de ladite tige (3), et/ou dans lequel ledit composant prosthétique (1) est un composant tibial d'une prothèse de genou et dans lequel ledit dispositif (10) comprend une base (12) ayant la forme sensiblement en C et dotée d'une surface supérieure (12a) et d'une surface inférieure (12d), cette dernière étant adaptée pour être maintenue et/ou pour entrer en contact pendant l'utilisation avec l'os du patient, ainsi qu'avec deux extrémités libres (12b, 12c) de ladite forme sensiblement en C, dirigée pendant l'utilisation vers l'arrière du genou ou dans lequel ledit composant prosthétique (1) est un composant fémoral d'une prothèse de hanche ou un composant huméral d'une prothèse d'épaule ou un composant d'une prothèse de coude, et ledit dispositif (10) comprend une base bridée (12) dotée d'une surface supérieure (12a) et d'une surface inférieure (12d), cette dernière étant adaptée pour être maintenue et/ou pour entrer en contact pendant l'utilisation avec l'os du patient.

3. Kit selon l'une quelconque des revendications précédentes, dans lequel ledit composant prosthétique (1) comprend une plaque tibiale (2) et dans lequel ladite base (12) est adaptée pendant l'utilisation pour entrer en contact et/ou pour être maintenue avec une face inférieure (2a) de ladite plaque tibiale (2) et/ou a une forme et une taille correspondant sensiblement à celles de ladite plaque tibiale (2) et/ou a un évidement (12e) qui détermine ladite forme sensiblement en C et sépare lesdites extrémités libres (12b, 12c).

4. Kit selon l'une quelconque des revendications précédentes, dans lequel ledit dispositif (10) comprend un arbre (13) doté d'une partie (13a) ayant une forme sensiblement tubulaire ou conique ou frustoconique ou frustopyramidale et une section prise le long d'un plan parallèle au plan transversal du corps humain qui est sensiblement circulaire ou ovale, ou carrée avec des bords adoucis, ou polygonale avec des bords adoucis, etc., dans lequel ledit arbre (13) part d'une surface inférieure (12d) de ladite base (12), dans une direction sensiblement perpendiculaire à ladite base (12) ou inclinée par rapport à ladite base (12), de façon à être inséré dans le canal médullaire de l'os du patient et/ou dans lequel ledit arbre (13) est adapté pendant l'utilisation pour contenir ladite tige (3) dudit composant prosthétique (1) et/ou dans lequel ledit arbre (13) a des dimensions légèrement supérieures à celles de ladite tige (3).

5. Kit selon l'une quelconque des revendications précédentes, dans lequel ledit composant prosthétique a au moins deux ailes latérales (3b, 3c) et dans lequel ladite partie (13a) dudit arbre (13) comprend au moins deux protubérances latérales (13b, 13c), ayant éventuellement une forme sensiblement triangulaire, par exemple un triangle à angle droit, et/ou une forme et une position correspondant sensiblement à celle desdites au moins deux ailes latérales (3b, 3c) mais avec des dimensions légèrement supérieures à la taille desdites au moins deux ailes latérales (3b, 3c), de telle manière que lesdites au moins deux protubérances latérales (13b, 13c) puissent pendant l'utilisation, à l'intérieur de celles-ci, contenir lesdites au moins deux ailes latérales (3b, 3c) .

6. Kit selon l'une quelconque des revendications précédentes, dans lequel ladite ouverture (15) est adaptée pour permettre l'insertion dudit composant prosthétique et/ou de ladite tige (3) et/ou desdites au moins deux ailes latérales (3b, 3c), dans lequel ladite ouverture (15) affecte au moins ladite base (12) et ladite partie (13a) dudit dispositif (10).

7. Kit selon la revendication précédente, dans lequel ladite ouverture (15) sous-tend ladite cavité (16) présente dans ledit dispositif (10), dans lequel ladite ouverture (15) comprend une zone (15a), ayant une forme sensiblement circulaire ou polygonale et des dimensions légèrement supérieures à celles de la section transversale de ladite portion (3a) de ladite tige (3) et/ou au moins deux ouvertures latérales (15b, 15c) adaptées pendant l'utilisation pour insérer lesdites au moins deux ailes latérales (3b, 3b) de ladite tige (3) et/ou dans lequel ladite cavité (16) a, dans ladite zone (15a), un espace (16a), ayant une forme sensiblement cylindrique ou conique ou frustoconique ou frustopyramidale et des dimensions légèrement supérieures à celles de ladite portion (3a) de ladite tige (3) qui est contenue pendant l'utilisation dans ledit espace (16a) et/ou, à lesdites ouvertures (15b, 15c), au moins deux portions latérales creuses (16b, 16c), qui s'étend dans lesdites protubérances latérales (13b, 13c) dudit arbre (13) dudit dispositif (10).

8. Kit selon l'une quelconque des revendications précédentes, dans lequel ledit arbre (13) a pendant l'utilisation une progression latérale inclinée ou incurvée ou légèrement inclinée pendant l'utilisation vers l'arrière du genou et/ou comprend une base de fermeture (17) de ladite partie (13a) dudit arbre (13), dans lequel ladite base de fermeture (17) est inclinée vers le haut à l'arrière par rapport à la base (12) dudit dispositif (10).

9. Kit selon l'une quelconque des revendications précédentes, dans lequel ledit dispositif (10) est poreux et/ou doté de trous traversants, pouvant pendant l'utilisation déplacer le ciment osseux de l'intérieur dudit dispositif (10) vers l'extérieur de celui-ci et/ou vers l'os du patient, ledit ciment osseux étant éventuellement en phase liquide ou fluide, adapté pour fixer ledit composant prosthétique (1) à l'os du patient.

10. Kit selon l'une quelconque des revendications précédentes, dans lequel ledit composant prosthétique (1) est ledit composant tibial d'une prothèse de genou et ladite plaque tibiale (2) s'étend selon une progression sensiblement parallèle à un plan transversal du corps humain qui passe à l'extrémité proximale ou l'épiphyse proximale du tibia et comprend ladite face inférieure (2a) et une face supérieure (2d), opposée à ladite face inférieure (2a) et dirigée vers un composant fémoral d'une prothèse de genou et/ou dans lequel ladite plaque tibiale (2) a une forme sensiblement en C, deux extrémités libres (2b, 2c) de ladite forme sensiblement en C étant placées pendant l'utilisation à l'arrière du genou et un évidement (2e) étant placé entre lesdites extrémités libres (2b, 2c).

11. Kit selon l'une quelconque des revendications précédentes, dans lequel ladite tige (3) comprend une portion sensiblement cylindrique (3a) s'étendant depuis ladite face inférieure (2a) de ladite plaque tibiale (2) et/ou dans lequel ladite tige (3) et/ou ladite portion (3a) s'étend au canal médullaire du tibia du patient.

12. Kit selon l'une quelconque des revendications précédentes, dans lequel ladite tige (3) comprend au moins deux ailes latérales (3b, 3c), dans lequel lesdites au moins deux ailes latérales (3b, 3c) partent de ladite portion (3a) et ont une forme sensiblement triangulaire, par exemple avec un angle droit et/ou ont chacune un côté long qui adhère et/ou est sensiblement maintenu à l'extension verticale pendant l'utilisation de ladite première portion (3a), un côté court qui adhère et/ou est sensiblement maintenu sur ladite face inférieure (2a) de ladite plaque tibiale (2) et une diagonale s'étendant vers l'extérieur par rapport à ladite première portion (3a) de ladite tige (3).

13. Kit selon la revendication précédente, dans lequel chaque côté court desdites au moins deux ailes latérales (3b, 3c) s'étend vers lesdites extrémités libres (2b, 2c) de ladite plaque tibiale (2) et/ou dans lequel lesdites au moins deux ailes latérales (3b, 3c) déterminent un angle entre elles, pendant l'utilisation à la partie arrière du genou, inférieur à 180° ou compris entre 45° et 135°.

14. Kit selon l'une quelconque des revendications précédentes, dans lequel ledit composant prosthétique (1) est un composant fémoral d'une prothèse de genou ou un composant huméral d'une prothèse d'épaule, ou un composant d'une prothèse de coude, dans lequel ledit composant prosthétique comprend une tête sphérique ou semi-sphérique ou en forme de capuchon sphérique (20), et une structure semblable à un col (21) ayant une forme sensiblement cylindrique ou frustoconique ou frustopyramidale et adaptée pour raccorder ladite tige (3) et ladite tête (20), dans lequel ladite tige (3) a une extrémité de raccordement (23) adaptée pendant l'utilisation pour être raccordée et/ou maintenue sur ladite structure semblable à un col (21), dans lequel ladite extrémité de raccordement (23) part de ladite structure semblable à un col (21) vers l'extérieur et a une forme frustoconique ou frustopyramidale, jusqu'à ladite tige (3) où une bride périphérique (23a) est présente s'étendant vers l'extérieur dudit composant prosthétique (1) et dotée d'une face inférieure (23b).

15. Kit selon l'une quelconque des revendications précédentes, dans lequel ledit dispositif (10) est préformé.

16. Kit selon l'une quelconque des revendications précédentes, dans lequel ledit composant prosthétique (1) comprend des sièges de logement (18) pour des moyens de raccordement (19) correspondants présent dans ledit dispositif (10), ou vice versa, dans lequel lesdits sièges de logement (18) sont formés comme des ouvertures traversantes ou des ouvertures non traversantes s'étendant le long d'un axe parallèle à l'axe longitudinal du corps humain et/ou pendant l'utilisation dans une direction verticale dans l'épaisseur de ladite plaque tibiale (2) et dotés d'au moins une ouverture sur ladite face inférieure (2a) de ladite plaque tibiale (2) ou de ladite bride périphérique (23a) alors que lesdits moyens de raccordement (19) adaptés pendant l'utilisation pour être contenus dans lesdits sièges de logement (18), ont une forme sensiblement de goupille, cylindre, semi-cylindre, clip, C, annulaire ou tubulaire, etc., et sont positionnés sur ladite base (12) et/ou sur ladite surface supérieure (12a) de ladite base (12).

17. Dispositif en forme de boîtier ou de coque (10) pour un composant prosthétique (1), comme par exemple un composant tibial et/ou fémoral d'une prothèse de genou adaptée pour être implantée à une extrémité de l'os tibial et/ou de l'os fémoral du patient sur une articulation de genou, ou un composant fémoral d'une prothèse de hanche, adapté pour être implanté pendant l'utilisation à une extrémité du l'os fémoral du patient, ou un composant huméral d'une prothèse d'épaule, ou un composant d'une prothèse de coude, dans lequel ledit composant prosthétique est constitué d'un matériau métallique, dans lequel ledit composant prosthétique est selon au moins l'une des revendications 1 à 16, dans lequel ledit dispositif en forme de boîtier ou de coque (10) est adapté pour être implanté pendant l'utilisation sur un os d'un patient, dans lequel ledit dispositif (10) comprend une ouverture (15) et une cavité (16) et est adapté pendant l'utilisation pour recouvrir extérieurement et/ou pour contenir, à l'intérieur de celui-ci, au moins une tige (3) dudit composant prosthétique (1), dans lequel ledit dispositif (10) est placé pendant l'utilisation entre ledit composant prosthétique et l'os du patient, de manière à empêcher pendant l'utilisation le contact direct entre le composant prosthétique (1) et l'os du patient et à maintenir de manière stable ledit composant prosthétique sur l'os du patient, **caractérisé en ce que** ledit dispositif (10) est constitué d'une matière plastique, comme le ciment osseux et/ou PMMA.

18. Dispositif selon la revendication 17, dans lequel ledit dispositif (10) a une forme correspondant sensiblement à celle dudit composant prosthétique (1) et/ou de ladite tige (3) dudit composant prosthétique (1), et/ou dans lequel ledit dispositif (10) comprend une base (12) ayant la forme sensiblement en C et est doté d'une surface supérieure (12a) et d'une surface inférieure (12d), ainsi que de deux extrémités libres (12b, 12c) de ladite forme sensiblement en C, dirigées pendant l'utilisation vers l'arrière du genou, dans lequel ladite base (12) est adaptée pendant l'utilisation pour entrer en contact et/ou être maintenue avec une face inférieure (2a) de ladite plaque tibiale (2) et/ou a une forme et une taille correspondant sensiblement à celles de ladite plaque tibiale (2) et/ou a un évidement (12e) qui détermine ladite forme sensiblement en C et sépare lesdites extrémités libres (12b, 12c) ou dans lequel ledit dispositif (10) comprend une base bridée (12) dotée d'une surface supérieure (12a) et d'une surface inférieure (12d), cette dernière étant adaptée pour être maintenue sur et/ou pour entrer en contact pendant l'utilisation avec l'os du patient.

19. Dispositif selon l'une quelconque des revendications 17 ou 18, dans lequel ledit dispositif (10) comprend un arbre (13) doté d'une partie (13a) ayant une forme sensiblement tubulaire ou cylindrique ou conique ou frustoconique ou frustopyramidale, et une section prise le long d'un plan parallèle au plan transversal du corps humain qui est sensiblement circulaire ou ovale, ou carrée avec des bords adoucis, ou polygonale avec des bords adoucis, etc., dans lequel ledit arbre (13) part d'une surface inférieure (12d) de ladite base (12), dans une direction sensiblement perpendiculaire ou inclinée par rapport à ladite base (12), de manière à être insérée pendant l'utilisation dans le canal médullaire de l'os du patient et/ou dans lequel ledit arbre (13) est adapté pendant l'utilisation pour contenir ladite tige (3) dudit composant prosthétique (1) et/ou dans lequel ledit arbre (13) a des dimensions légèrement supérieures à celles de ladite tige (3).

20. Dispositif selon l'une quelconque des revendications 17 à 19, dans lequel ladite partie (13a) comprend au moins deux protubérances latérales (13b, 13c), ayant éventuellement une forme sensiblement triangulaire, par exemple le triangle à angle droit, et/ou une forme et une position qui correspondent sensiblement à celles d'au moins deux ailes latérales (3b, 3c) dudit composant prosthétique (1) mais avec des dimensions légèrement supérieures à celles desdites au moins deux ailes latérales (3b, 3c), de telle manière que lesdites au moins deux protubérances latérales (13b, 13c) puissent contenir pendant l'utilisation, à l'intérieur de celles-ci, lesdites au moins deux ailes latérales (3b, 3c).

21. Dispositif selon l'une quelconque des revendications 17 à 20, dans lequel ladite ouverture (15) est adaptée pour permettre l'insertion dudit composant prosthétique et/ou de ladite tige (3) et/ou desdites au moins deux ailes latérales (3b, 3c), dans lequel ladite ouverture (15) affecte au moins ladite base (12) et ladite partie (13a) dudit dispositif (10), dans lequel ladite ouverture (15) sous-tend ladite cavité (16) présente dans ledit dispositif (10), dans lequel ladite ouverture (15) comprend une zone (15a), ayant une forme sensiblement circulaire et/ou des dimensions légèrement supérieures à celles de la section transversale de ladite portion (3a) et/ou au moins deux ouvertures latérales (15b, 15c), adaptées pendant l'utilisation pour insérer lesdites au moins deux ailes latérales (3b, 3b) de ladite tige (3) et/ou dans lequel ladite cavité (16) a, à ladite zone (15a), un espace (16a) ayant une forme sensiblement cylindrique ou conique ou frustoconique ou frustopyramidale et/ou des dimensions légèrement supérieures à celles de ladite portion (3a) qui est contenue pendant l'utilisation dans ledit espace (16a) et/ou, à lesdites ouvertures (15b, 15c), au moins deux portions latérales creuses (16b, 16c) qui s'étend dans lesdites protubérances latérales (13b, 13c) dudit arbre (13) dudit dispositif (10).

22. Dispositif selon l'une quelconque des revendications 17 à 21, dans lequel ledit arbre (13) a une progression latérale légèrement inclinée, éventuellement pendant l'utilisation inclinée vers l'arrière du genou, et comprend une base de fermeture (17) de ladite partie (13a), dans lequel ladite base de fermeture (17) est inclinée, sur la partie arrière, vers le haut par rapport à la base (12) dudit dispositif (10) et/ou dans lequel ledit dispositif (10) est poreux et/ou doté de trous traversants, pouvant pendant l'utilisation déplacer le ciment osseux de l'intérieur dudit dispositif (10) vers l'extérieur de celui-ci et/ou vers l'os du patient, ledit ciment osseux étant éventuellement en phase liquide ou fluide, adapté pour fixer ledit composant prosthétique (1) sur l'os du patient.
